# EUROPÄISCHE PATENTANMELDUNG

(11) **EP 4 162 970 A1**
(43) Veröffentlichungstag der Anmeldung: **12.04.2023**
(21) Anmeldenummer: 22199704.2
(22) Anmeldetag: 05.10.2022
(51) Int. Cl.: A61M 35/00, A61K 35/02, A61K 39/35, A61J 9/00

(54) **STAUB-APPLIKATORVORRICHTUNG**

(30) Priorität: 06.10.2021 EP 21201088
(71) Anmelder: Hipp & Co, 6072 Sachseln (CH)
(72) Erfinder: KRETZSCHMAR, Sven, 85276 Pfaffenhofen (DE)
(74) Vertreter: Schiweck Weinzierl Koch Patentanwälte Partnerschaft mbB

(57) **Zusammenfassung**

Staub-Applikatorvorrichtung (1), welcher derart ausgebildet ist, insbesondere einen aus einem landwirtschaftlichen Betrieb gewonnenen landwirtschaftlichen Staub, welcher zur Behandlung von Allergien geeignet ist, aufzunehmen und anschließend freizusetzen, aufweisend: eine Staubaufnahme- und Freisetzeinrichtung (2) zum Aufnehmen und Freisetzen des landwirtschaftlichen Staubs, mit einer Aufnahmekammer zum Aufnehmen des landwirtschaftlichen Staubs aufweist, wobei die Aufnahmekammer staubdurchlässig ausgebildet ist für ein Herausrieselen des Staubs aus der Aufnahmekammer, und/oder wobei die Staubaufnahme- und Freisetzeinrichtung (2) wenigstens eine Aufnahmelage mit einer Aufnahmefläche auf der Außenseite zum Aufnehmen des landwirtschaftlichen Staubs (11) aufweist derart, dass zumindest der auf der Außenseite der Aufnahmefläche aufbringbare landwirtschaftliche Staub von der Aufnahmelage herausrieselbar ist, wobei die Staub-Applikatorvorrichtung (1) wenigstens eine Befestigungseinrichtung (3) aufweist oder die Staub-Applikatorvorrichtung integral oder einstückig mit einem Besteck (27), einem Gefäß, einem Babyfläschchen (4), einem Spielzeug, einem Schnuller oder einem Beruhigungssauger ausgebildet ist.

## Beschreibung

Die Erfindung betrifft eine Staub-Applikatorvorrichtung zur Applikation von insbesondere landwirtschaftlichem Staub, einschließlich eines Extrakts aus dem landwirtschaftlichen Staub, an Babys, Kleinkinder, Heranwachsende und Erwachsene, welcher besonders zur prophylaktischen und/oder therapeutischen Behandlung von Allergien bei Babys, Kleinkindern, Heranwachsenden und Erwachsenen geeignet ist.

Heutzutage leiden immer mehr Menschen an Allergien. Dieses sind Abwehrreaktionen des Immunsystems gegen eigentlich harmlose Stoffe, wie z.B. Hausstaub, Pollen, Tierhaare oder Nahrungsmittel.

In der WO 01/49319 A1 ist eine Zusammensetzung offenbart, die mindestens ein aerogen übertragbares mikrobielles Antigen umfasst, das typischerweise in Stallluft auftritt. Die Zusammensetzung wird dabei mit einem handelsüblichen Inhalator, Vernebler oder Beatmungsgerät verabreicht.

Aus der US 2014/1154290 A1 ist eine antiallergene Zusammensetzung bekannt, die mindestens ein Arabinogalactan- oder Arabinogalactan-Protein und/oder Huminsäure umfasst. Die Zusammensetzung wird in der Innenraumluft verteilt, um die Luft einer natürlichen landwirtschaftlichen Umgebung zu simulieren. Zum Verteilen der Zusammensetzung in der Innenraumluft wird die Zusammensetzung über einen Raumluft-Verteiler verteilt. Dieser verwendet ein Treibmittel wie Butan, Propan oder Druckluft.

Vor diesem Hintergrund ist die Aufgabe der Erfindung, eine Vorrichtung bereitzustellen welche eine vereinfachte Applikation insbesondere eines landwirtschaftlichen Staubs erlaubt, der zur Behandlung von Allergien geeignet ist, für die prophylaktische und/oder therapeutische Behandlung von Allergien bei einem Menschen, insbesondere einem Baby, einer Schwangeren oder einem Kleinkind. Insbesondere eine Vorrichtung, bei welcher eine treibmittellose Applikation eines landwirtschaftlichen Staubs, erlaubt wird, der insbesondere zur Behandlung von Allergien geeignet ist.

Die Aufgabe der vorliegenden Erfindung wird gelöst durch eine Staub-Applikatorvorrichtung gemäß den vorliegenden Ausführungen uns insbesondere gemäß den Ansprüchen.

Dabei wird eine Staub-Applikatorvorrichtung bereitgestellt, welche derart ausgebildet ist, insbesondere einen aus einem landwirtschaftlichen Betrieb gewonnenen landwirtschaftlichen Staub, welcher zur Behandlung von Allergien geeignet ist, aufzunehmen und anschließend freizusetzen zur Applikation des landwirtschaftlichem Staubs an Babys, Kleinkinder, Heranwachsende und Erwachsene, aufweisend:
eine Staubaufnahme- und Freisetzeinrichtung zum Aufnehmen und Freisetzen eines zur Behandlung von Allergien geeigneten landwirtschaftlichen Staubes, wobei die Staubaufnahme- und Freisetzeinrichtung zum Freisetzen mit dem landwirtschaftlichen Staub beaufschlagbar ist und wenigstens eine Aufnahmekammer mit einem Hohlraum zum Aufnehmen des landwirtschaftlichen Staubs aufweist, wobei die Aufnahmekammer für den in die Aufnahmekammer einfüllbaren Staub, insbesondere landwirtschaftlichen Staub, staubdurchlässig ausgebildet ist für ein Herausrieseln, insbesondere ein passives oder selbständiges Herausrieseln des Staubs aus der Aufnahmekammer,
und/oder wobei die Staubaufnahme- und Freisetzeinrichtung wenigstens eine Aufnahmelage mit einer Aufnahmefläche auf ihrer Außenseite zum Aufnehmen des landwirtschaftlichen Staubs aufweist derart, dass zumindest der auf der Außenseite der Aufnahmefläche aufbringbare Staub, insbesondere landwirtschaftliche Staub, von der Aufnahmelage herausrieselbar, insbesondere passiv oder selbständig herausrieselbar ist, wobei die Staub-Applikatorvorrichtung wenigstens ein oder zwei Befestigungseinrichtungen aufweist oder die Staub-Applikatorvorrichtung integral oder einstückig mit einem Besteck, einem Gefäß, einem Babyfläschchen, einem Spielzeug, einem Schnuller oder einem Beruhigungssauger ausgebildet ist.

Bei den benannten Allergien handelt es sich vorzugsweise um Heuschnupfen, und besonders bevorzugt um den fließenden Schnupfen bei Heuschnupfen. Die therapeutische und/oder prophylaktische Behandlung der Allergie bezieht sich dabei auf die Behandlung der mit der Allergie assoziierten Symptome, insbesondere um den fließenden Schnupfen bei Heuschnupfen.

Die Staub-Applikatorvorrichtung kann gleichwertig auch zur nicht-therapeutischen Applikation von landwirtschaftlichem Staub, welcher zur Behandlung von Allergien geeignet ist, an gesunde Babys, Kleinkinder, Heranwachsende und Erwachsene verwendet werden. "Gesund" bezeichnet dabei den Zustand, bei dem das entsprechende Baby, Kleinkind, der/die Heranwachsende und/oder der/die Erwachsene keine akuten Symptome einer Allergie aufweist, insbesondere keinen fließenden Schnupfen.

Die Staub-Applikatorvorrichtung hat den Vorteil, dass durch sie sehr einfach und kostengünstig eine Person und deren Immunsystem mit zur Behandlung von Allergien geeignetem landwirtschaftlichem Staub in Kontakt bringbar ist, ohne dass die Person sich dazu selbst in einem landwirtschaftlichen Betrieb oder einer landwirtschaftlichen Umgebung unmittelbar aufhalten muss. Durch den Kontakt des Immunsystems, insbesondere des Immunsystems eines Kindes, mit solchem landwirtschaftlichen Staub kann der Entwicklung einer Allergie, z.B. einer Pollenallergie oder Asthma, entgegengewirkt werden. Dies ist besonders vorteilhaft, wenn das Baby und seine Eltern nicht die Möglichkeit haben, sich in einer landwirtschaftlichen Umgebung aufzuhalten, insbesondere über eine längere Zeit.

In einer Ausführungsform der Erfindung weist die Aufnahmekammer ein Gehäuse mit einem Hohlraum auf, welches wenigstens eine oder mehrere Staubaustrittsöffnungen aufweist.

In einer weiteren Ausführungsform der Erfindung weist die Aufnahmekammer wenigstens ein oder mehrere ineinander geschachtelte Säckchen mit einem jeweiligen Hohlraum auf, wobei das jeweilige Säckchen staubdurchlässig ausgebildet ist. Das staubdurchlässige Säckchen ist beispielsweise aus einem staubdurchlässigen Material, einer staubdurchlässigen Materialkombination, einem staubdurchlässig-perforierten Material und/oder einer staubdurchlässig-perforierten Materialkombination hergestellt oder weist dieses auf.

In einer anderen Ausführungsform der Erfindung ist die Staubaufnahme- und Freisetzeinrichtung als aktive, treibmittellose Staubaufnahme- und Freisetzeinrichtung ausgebildet, wobei das Gehäuse oder das jeweilige Säckchen als Aufnahmekammer mit dem Hohlraum elastisch oder flexibel ausgebildet ist, derart, dass das Gehäuse mit seinem Hohlraum bzw. das jeweilige Säckchen mit seinem Hohlraum zusammendrückbar ist und in seine Ausgangsform zurückfedern oder zurückkehren oder im Wesentlichen zurückfedern oder zurückkehren kann, wobei der in dem Hohlraum des Gehäuses bzw. in dem Hohlraum des Säckchens einfüllbare Staub, insbesondere landwirtschaftliche Staub, dabei aus dem Gehäuse bzw. dem Säckchen nach außen freisetzbar ist.

In einer Ausführungsform der Erfindung ist unter der Aufnahmelage wenigstens eine weitere Aufnahmelage vorgesehen, wobei zwischen den beiden Aufnahmelagen zusätzlich ein Staub, insbesondere landwirtschaftlicher Staub, einbringbar ist und wobei die Aufnahmelagen hierzu für den zwischen die beiden Aufnahmelagen einbringbaren landwirtschaftlichen Staub zusätzlich staubdurchlässig und/oder staubdurchlässig perforiert sind derart, dass der Staub nach außen austreten kann, insbesondere passiv oder selbständig austreten kann.

In einer weiteren Ausführungsform der Erfindung besteht wenigstens eine Aufnahmelage aus einem Stoffgewebe, besonders einem glatten Stoffgewebe, einem Florgewebe oder einem aufgerauten Stoffgewebe, einem Metallgewebe, wenigstens einer Papierschicht, wenigstens einer Folienschicht, insbesondere einer Metallfolienschicht und/oder einer Kunststofffolienschicht, wenigstens einer Filterpapierschicht, einer Schaumstoffschicht und/oder wenigstens einer Schwamm- oder Schaumstoffschicht.

Gemäß einer Ausführungsform der Erfindung ist die Staubaufnahme- und Freisetzeinrichtung als aktive, treibmittellose Staubaufnahme- und Freisetzeinrichtung ausgebildet, wobei die Aufnahmelage oder Aufnahmelagen elastisch oder flexibel sind derart, dass die jeweilige Aufnahmelage zusammendrückbar ist und in ihre Ausgangsform zurückfedern oder zurückehren oder im Wesentlichen zurückfedern oder zurückkehren kann, wobei der landwirtschaftliche Staub hierbei nach außen freisetzbar ist.

In einer Ausführungsform der Erfindung weist die Staub-Applikatorvorrichtung wenigstens eine Befestigungseinrichtung zum Befestigen der Staub-Applikatorvorrichtung auf, z.B. an einem Gefäß, beispielsweise Trinkgefäß (und/oder dessen Verschluss wie zB einem Sportscap), insbesondere einem Babyfläschchen, einem Strohhalm, einem Besteck (z.B. Löffel, Gabel, Schieber etc) einem Kleidungsstück, einer Decke, einem Spielzeug, einem Schnuller einem Beruhigungssauger und/oder einem Kissen, insbesondere Stillkissen.

Gemäß einer Ausführungsform der Erfindung weist die Befestigungseinrichtung wenigstens eine Klebeschicht, eine Klebeschicht z.B. optional mit einer zusätzlichen abziehbaren Träger- und Trennschicht, eine Anstecknadel, eine Befestigungsöse, einen Befestigungshaken, einen Befestigungsclip, ein Band, insbesondere Gummiband, ein Stoffband, ein Papierband, oder ein Klettband usw., eine Lasche, insbesondere Stofflasche oder Gummilasche usw., und/oder eine Magnetbefestigungseinrichtung auf.

In einer anderen Ausführungsform der Erfindung weist die Staub-Applikatorvorrichtung ein Trägerelement als Träger für die Staubaufnahme- und Freisetzeinrichtung auf. Das Trägerelement ist dabei z.B. einstückig mit der Befestigungseinrichtung ausgebildet oder an einem Teil der Befestigungseinrichtung als separates Teil befestigt, z.B. angeklebt.

Gemäß einer Ausführungsform der Erfindung besteht wenigstens ein Bereich, insbesondere wenigstens eine Seite des jeweiligen Säckchens aus wenigstens einer Lage aus einem insbesondere für einen zur Behandlung von Allergien geeigneten landwirtschaftlichen Staub staubdurchlässigen oder staubdurchlässig-perforierten Material oder einer für den zur Behandlung von Allergien geeigneten landwirtschaftlichen Staub staubdurchlässigen oder staubdurchlässig-perforierten Materialkombination zur Freisetzung des landwirtschaftlichen Staubs nach außen aus der Staub-Applikatorvorrichtung.

In einer anderen Ausführungsform der Erfindung weist das Gehäuse einen Gehäusedeckel auf zum Öffnen und Schließen des wenigstens einen mit dem landwirtschaftlichen Staub versehbaren Hohlraums des Gehäuses. Der Gehäusedeckel ist z.B. vollständig abnehmbar oder über wenigstens ein Scharnier schwenkbar mit dem Gehäuse verbunden ausgebildet. Der Gehäusedeckel ist dabei z.B. geschlossen ausgebildet oder mit wenigstens einer oder mehreren Staubaustrittsöffnungen versehen.

In einer weiteren Ausführungsform der Erfindung weist die Staub-Applikatorvorrichtung ein Deckelelement, insbesondere ein teilweise oder vollständig abnehmbares Deckelelement, auf zum Abdecken zumindest der Staubaufnahme- und Freisetzeinrichtung, wobei das Deckelelement geschlossen oder mit einer oder mehreren Staubaustrittsöffnungen versehen ist. Das Deckelelement ist formstabil oder derart elastisch oder flexibel ausgebildet, dass es zusammendrückbar und anschließend in seine Ausgangsform zurückfedert oder zurückkehrt oder im Wesentlichen zurückfedert oder zurückkehrt.

Gemäß einer Ausführungsform der Erfindung ist wenigstens die Staubaufnahme- und Freisetzeinrichtung mit einer Schutzhülle versehbar ist, wobei die Schutzhülle z.B. aus Kunststofffolie, insbesondere transparenter Kunststofffolie, oder Papier ist.

In einer weiteren Ausführungsform der Erfindung ist die Staub-Applikatorvorrichtung an einem Schnuller oder einem Beruhigungssauger in diesen integriert oder bildet einen Teil davon.

Weitere Einzelheiten, Merkmale und Vorteile der Erfindung ergeben sich aus den Zeichnungen sowie aus der nachfolgenden Beschreibung von bevorzugten Ausführungsformen anhand der Zeichnungen. Die Zeichnungen illustrieren dabei lediglich beispielhafte Ausführungsformen der Erfindung, welche den wesentlichen Erfindungsgedanken nicht einschränken.
In Figur 1 ist eine Staub-Applikatorvorrichtung gemäß einer ersten Ausführungsform der Erfindung in einer teilweisen geschnitten, Perspektivansicht gezeigt;
In Figur 2 ist die Staub-Applikatorvorrichtung gemäß Figur 1 in einer Schnittansicht gezeigt;
In Figur 3 ist die Staub-Applikatorvorrichtung gemäß Figur 1 und 2 in einer Vorderansicht und an einer Babyflasche befestigt gezeigt;
In Figur 4 ist ein weiteres Ausführungsbeispiel der Staub-Applikatorvorrichtung gemäß der Figuren 1 bis 3 in einer teilweisen Schnittansicht gezeigt;
In Figur 5 ist eine Staub-Applikatorvorrichtung gemäß einer zweiten Ausführungsform der Erfindung in einer Perspektivansicht gezeigt;
In Figur 6 ist eine Staub-Applikatorvorrichtung gemäß einer dritten Ausführungsform der Erfindung in einer Perspektivansicht gezeigt;
In Figur 7 ist die Staub-Applikatorvorrichtung gemäß Figur 6 gedreht und in einer Schnittansicht gezeigt;
In Figur 8 ist eine Staub-Applikatorvorrichtung gemäß einer vierten Ausführungsform der Erfindung in einer Perspektivansicht gezeigt;
In Figur 9 ist die Staub-Applikatorvorrichtung gemäß Figur 8 in einer teilweisen Schnittansicht gezeigt;
In Figur 10 ist die Staub-Applikatorvorrichtung gemäß Figur 8 in einer teilweisen Schnittansicht gezeigt, bei welcher das zusätzliche Deckelement geschnitten dargestellt ist; und
In Figur 11 ist ein Ausführungsbeispiel zum Vorsehen der erfindungsgemäßen Staub-Applikatorvorrichtung an einem Löffel gezeigt.

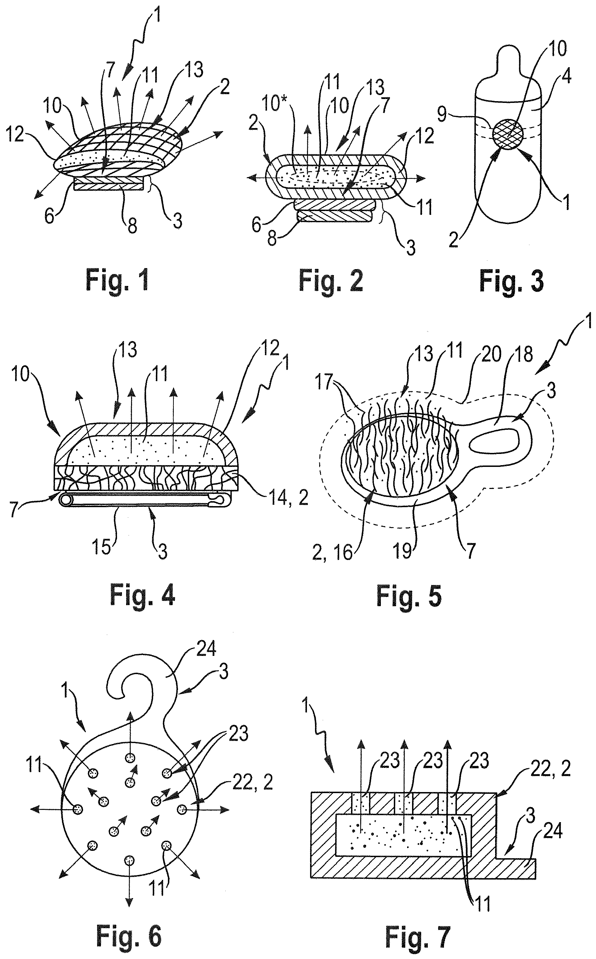

In Figur 1 ist eine schematische und stark vereinfachte Ansicht einer Staub-Applikatorvorrichtung 1 gemäß einer ersten Ausführungsform der Erfindung in einer teilweisen geschnitten, gezeigten Perspektivansicht dargestellt. In Figur 2 ist die Staub-Applikatorvorrichtung 1 gemäß Figur 1 in einer Schnittansicht und in Figur 3 in einer Vorderansicht, und beispielsweise dabei an einer Babyflasche befestigt gezeigt.

Die Staub-Applikatorvorrichtung 1 weist dabei eine Staubaufnahme- und Freisetzeinrichtung 2 auf.

Die Staub-Applikatorvorrichtung 1 gemäß der Erfindung ist derart ausgebildet, vorzugsweise ein von einem landwirtschaftlichen Betrieb gewonnen landwirtschaftlichen Staub, welcher zur Behandlung von Allergien geeignet ist, aufzunehmen und anschließend freizusetzen. Dies gilt für alle Ausführungsformen der Erfindung. Bei dem hierin verwendbaren Staub, genauer landwirtschaftlichen Staub, der erfindungsgemäßen Staub-Applikatorvorrichtung 1 handelt es sich vorzugsweise ausschließlich oder im Wesentlichen um aus einem landwirtschaftlichen Betrieb gewonnenen Staub, insbesondere Staub aus einem Viehstall, z.B. Kuhstall, welcher zur Behandlung von Allergien geeignet und hierfür geeignet aufbereitet ist. Daher wird in der Beschreibung der Begriff landwirtschaftlicher Staub verwendet. Der zur Behandlung von Allergien geeignete landwirtschaftliche Staub aus dem landwirtschaftlichen Betrieb kann hierbei beispielsweise aus abgesaugter Betriebsluft aus der Betriebsumgebung, z.B. dem Hof, des landwirtschaftlichen Betriebs, aus abgesaugter Betriebsluft aus einem jeweiligen Betriebsraum des landwirtschaftlichen Betriebs, z.B. dem Stall, insbesondere dem Viehstall, gewonnen und geeignet aufbereitet werden, z.B. gefiltert werden, ein oder mehrere Inhalte oder Inhaltsstoffe extrahiert werden usw.. Das zusätzliche Filtern kann z.B. vorgesehen werden, um beispielsweise unerwünschte Partikel oder Elemente aus dem angesaugten Staub herauszufiltern und/oder die Staubpartikelgröße auf eine vorbestimmte maximale Größe zu beschränken und so zu große Partikel herausfiltern usw.. Zusätzlich oder alternativ kann der landwirtschaftliche Staub auch aus einer oder mehreren abgesaugten Flächen in und/oder an einem Betriebsgebäude des landwirtschaftlichen Betriebs, gewonnen und wahlweise zusätzlich ebenfalls geeignet aufbereitet werden, z.B. gefiltert und/oder wenigstens ein Inhalt oder Inhaltsstoff extrahiert werden. Als Flächen zum Absaugen des landwirtschaftlichen Staubs können beispielsweise Gebäudeflächen des landwirtschaftlichen Gebäudes (z.B. Viehstall usw.) darunter Innen- und/oder Außenwände, Innen- und/oder Außen- Fensterflächen, Innen- und oder Außen- Fensterbänke, der Innenboden, Flächen des Stallinventars, wie Gitterflächen, Futtertrogflächen, Melkmaschinenenflächen usw. des landwirtschaftlichen Betriebs abgesaugt und der abgesaugte Staub gegebenenfalls zusätzlich nochmals gefiltert und/oder wenigstens ein Inhalt oder Inhaltsstoff daraus extrahiert werden. Die Erfindung ist jedoch auf die genannten Beispiele wie der Betriebsluft und insbesondere Stallluft, darunter Kuhstallluft, sowie den in und/oder an dem Betriebsgebäude des landwirtschaftlichen Betriebs befindlichen Flächen zum Absaugen und gegebenenfalls zusätzlichen Filtern nicht beschränkt. Besonders bevorzugt ist die erfindungsgemäße Staub-Applikatorvorrichtung 1 beispielsweise mit einem landwirtschaftlicher Staub, einschließlich eines Extrakts davon, der z.B. in der WO2020120724 beschrieben ist beaufschlagbar, insbesondere mit dem landwirtschaftlichem Staub gemäß den dort offenbarten Ansprüchen 19-24 oder hergestellt über das Verfahren der dort offenbarten Ansprüche 1-18. Vorstehende Ausführungen gelten für alle Ausführungsformen der Erfindung.

Der aus dem landwirtschaftlichen Betrieb und insbesondere dem landwirtschaftlichen Viehhaltungsbetrieb gewonnene landwirtschaftliche Staub, welcher zur Behandlung von Allergien geeignet ist, wird auf die Staub-Applikatorvorrichtung 1 aufgebracht und kann anschließend von der Staub-Applikatorvorrichtung 1 wieder freigesetzt werden, insbesondere passiv und/oder treibmittellos aktiv, beispielsweise in der Umgebung eines (beispielsweise in der Stadt lebenden) Babys, Kindes, Heranwachsenden oder Erwachsenen. Der Grundgedanke der Erfindung besteht darin, dass Kinder, die in der Stadt aufwachsen, häufig mehr Allergien entwickeln, darunter z.B. Pollenallergien und Asthma, als Kinder, die in einer landwirtschaftlichen Umgebung aufwachsen. Mittels der erfindungsgemäßen Staub-Applikatorvorrichtung 1, welche im Folgenden anhand von verschiedenen Ausführungsbeispielen beschrieben wird, kann beispielsweise das Immunsystem eines Babys, welches außerhalb der Reichweite von landwirtschaftlichem Staub (beispielsweise in der Stadt) aufwächst, sehr einfach bereits frühzeitig mit einem landwirtschaftlichen Staub, welcher zur Behandlung von Allergien geeignet ist, problemlos in Kontakt gebracht werden, ohne dass das Baby sich dafür umgekehrt längere Zeit und wiederholt immer wieder in einer landwirtschaftlichen Umgebung, wie z.B. einem Bauernhof, mit seinen Eltern aufhalten müsste. Dabei ist auch ein Einsatz von zusätzlichem Treibmittel zum Freisetzen des Staubs bei der erfindungsgemäßen Staub-Applikatorvorrichtung, im Gegensatz zu den Zusammensetzungen im Stand der Technik, nicht erforderlich.

Durch die erfindungsgemäße Staub-Applikatorvorrichtung 1 können somit insbesondere Babys, aber auch z.B. Schwangere, sowie Kleinkinder und deren Immunsystem durch einen mittels der Staub-Applikatorvorrichtung 1 freisetzbaren, zur Behandlung von Allergien geeigneten landwirtschaftlichen Staub sehr einfach in Kontakt gebracht werden und einer Entwicklung von Allergien, wie beispielsweise einer Pollenallergie, sowie Asthma, entgegengewirkt werden. Das Immunsystem kann somit mittels der erfindungsgemäße Staub-Applikatorvorrichtung 1 bereits frühzeitig gezielt trainiert werden, um der Entwicklung einer Allergie und Asthma im späteren Leben entgegenzuwirken.

Bei dem ladwirtschaftlichen Viehhaltungsbetrieb als Beispiel für einen landwirtschaftlichen Betrieb aus welchem der landwirtschaftliche Staub gewonnen werden kann, handelt es sich insbesondere um einen Bauernhof mit Vieh oder Viehhaltung, insbesondere mit wenigstens einer oder mehreren Kühen. Zusätzlich oder statt der Kühe kann der Bauernhof mit Vieh oder Viehhaltung auch z.B. wenigstens ein oder mehrere Pferde, Schweine, Schafe, Ziegen, Hühner usw. aufweisen. Bevorzugt handelt es sich bei dem landwirtschaftlichen Viehhaltungsbetrieb um einen Bio-Hof, insbesondere einem Bio-Hof der ökologische/biologische Erzeugnisse gemäß der Verordnung (EG) Nr. 834/2007 herstellt.

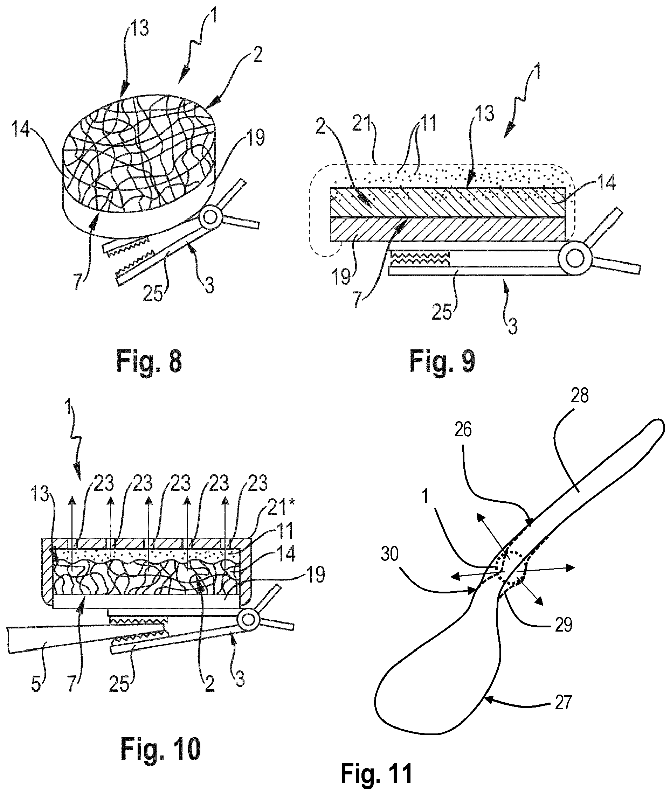

Bei dem in den Figuren 1 bis 3 gezeigten ersten Ausführungsbeispiel der erfindungsgemäßen Staub-Applikatorvorrichtung 1, weist die Staub-Applikatorvorrichtung 1 die Staubaufnahme- und Freisetzeinrichtung 2 und wenigstens eine optionale zusätzliche Befestigungseinrichtung 3 zum Befestigen der Staubaufnahme- und Freisetzeinrichtung 2, beispielsweise an einem Gegenstand wie einer Babyflasche 4, einem Besteck, wie z.B. einem Löffel, wie in nachfolgender Fig. 11 gezeigt, einer Decke, einem Spielzeug oder einem Kleidungsstück 5, z.B. einer Bluse oder Shirt wie in nachfolgender Figur 10, usw., auf. In dem in den Figuren 1 bis 3 gezeigten Beispiel ist als optionale Befestigungseinrichtung 3 z.B. eine Klebeschicht 6 an der Unterseite oder Befestigungsseite 7 der Staubaufnahme- und Freisetzeinrichtung 2 angebracht. Auf der Klebeschicht 6 ist zusätzlich eine abziehbare Träger- und Trennschicht 8, z.B. ein Trennpapier, angebracht, die vor dem Aufkleben der Staubaufnahme- und Freisetzeinrichtung 2 mittels der Klebeschicht 6 abgestreift wird. Anschließend kann die Staub-Applikatorvorrichtung 1 mit ihrer Staubaufnahme- und Freisetzeinrichtung 2 beispielsweise an der in Figur 3 stark vereinfachten und nicht maßstäblich dargestellten Babyflasche 4 angeklebt werden oder jedem anderen Artikel, wie z.B. einem Besteck, wie dem Babylöffel in Fig. 11, einer Decke, insbesondere Babydecke, einem Spielzeug, einem Kleidungsstück usw.. Zusätzlich oder alternativ zu der Klebeschicht 6 und der Träger- und Trennschicht 8 als Befestigungseinrichtung 3 kann aber auch jede andere Befestigungseinrichtung 3 vorgesehen werden zur Befestigung an einem zugeordneten Gegenstand. Wie in Figur 3 mit einer gestrichelten Linie angedeutet ist, kann auch beispielsweise ein Stoffband, Klettband oder Gummiband 9 usw. als Befestigungseinrichtung 3 an der Staubaufnahme- und Freisetzeinrichtung 2 angebracht sein, mit welcher die Staub-Applikatorvorrichtung 1 z.B. an einem Besteck, wie dem Löffel in Fig. 11, oder z.B. an einer Babyflasche 4 sehr leicht abnehmbar angebracht und dabei außerdem sehr einfach an z.B. verschiedene Flaschengrößen im Falle der Babyflasche angepasst werden kann.

Die Staubaufnahme- und Freisetzeinrichtung 2 ist in dem in den Figuren 1 bis 3 gezeigten Ausführungsbeispiel als Aufnahmekammer mit einem Hohlraum und genauer als staubdurchlässiges Säckchen 10 ausgebildet, insbesondere für einen mit ihm befüllbaren zur Behandlung von Allergien geeigneten landwirtschaftlichen Staub 11. Das staubdurchlässige Säckchen 10 als Aufnahmekammer ist aus einem staubdurchlässigen Material, einer staubdurchlässigen Materialkombination, einem staubdurchlässig-perforierten Material und/oder einer staubdurchlässig-perforierten Materialkombination.

Das Säckchen 10 weist dabei wenigstens eine nicht dargestellte Befüllöffnung auf zum Befüllen des Säckchens 10 mit einem zur Behandlung von Allergien geeigneten landwirtschaftlichen Staub 11. Die Befüllöffnung kann dabei wahlweise zusätzlich verschließbar und insbesondere wiederverschließbar ausgebildet sein. Beispielsweise kann die Befüllöffnung des Säckchens 10 mittels eines (nicht dargestellten) Verschlusselements, z.B. eines Klettverschlusses oder eines Klebestreifens usw., einmal verschließbar oder mehrfach verschließbar bzw. wiederverschließbar ausgebildet sein.

Das Austreten des landwirtschaftlichen Staubs 11 aus der Staub-Applikatorvorrichtung 1 nach außen ist in den Figuren 1, 2 und 3 sowie z.B. den nachfolgenden Figuren 4, 6, 7, 10 und 11 mit Pfeilen angedeutet.

Das Säckchen 10 kann als passive und vorzugsweise zusätzlich als treibmittellose, aktive Staubaufnahme- und Freisetzeinrichtung 2 in dem in den Figuren 1 bis 3 gezeigten Ausführungsbeispiel ausgebildet sein.

Als passive Staubaufnahme- und Freisetzeinrichtung 2 ist das Säckchen 10 staubdurchlässig ausgebildet, beispielsweise zumindest eine Staubaustrittsseite des Säckchens 10. Der in das Säckchen 10 eingefüllte Staub 11 kann somit von selbst aus dem staubdurchlässigen Säckchen 10 herausrieseln. Dies kann zusätzlich weiter unterstützt werden, wenn die Staub-Applikatorvorrichtung 1 mit ihrer Staubaufnahme- und Freisetzeinrichtung 2 in Form des Säckchens 10 an einem Gegenstand wie z.B. einer Babyflasche 4, einen löffel usw. mittels der zusätzlichen Befestigungseinrichtung 3 befestigt wird und zusammen mit der Babyflasche 4 z.B. beim Füttern des Babys bewegt wird.

Als eine treibmittellose, aktive Staubaufnahme- und Freisetzeinrichtung 2 ist das Säckchen 10 für den eingefüllten Staub staubdurchlässig und zusätzlich elastisch oder flexibel ausgebildet derart, dass der Hohlraum, den das Säckchen 10 bildet, zusammendrückbar ist und anschließend in seine Ausgangsform oder im Wesentlichen in seine Ausgangsform zurückkehren oder zurückfedern kann. Ein in das Säckchen 10 eingefüllter Staub 11 kann durch das Zusammendrücken des Säckchens 10 und des von ihm gebildeten Hohlraums durch das staubdurchlässige Säckchen 10 somit aktiv nach außen freigesetzt werden. Das hat den Vorteil, dass beispielsweise kein zusätzliches Treibmittel, wie Treibgas oder Druckluft, vorgesehen werden muss zum aktiven Freisetzen eines in das Säckchen 10 eingefüllten Staubs 11. Es reicht, das Säckchen 10 aktiv zu drücken, vorzugsweise wiederholt zusammenzudrücken.

Beispielsweise kann das Säckchen 10 aus wenigstens einem staubdurchlässigen Filz, einem staubdurchlässigen oder staubdurchlässig-perforierten Filterpapier, einem staubdurchlässigen Gewebe, insbesondere staubdurchlässigen Stoff- und/oder staubdurchlässigen Metallgewebe, wenigstens einer staubdurchlässig-perforierten Kunststofffolie, wenigstens einer staubdurchlässig-perforierten Metallfolie, wenigstens einer staubdurchlässig-perforierten Gummischicht und/oder einer staubdurchlässigen oder staubdurchlässig-perforierten Schwamm- und/oder Schaumstoffschicht ausgebildet sein zum Bereitstellen der Staubdurchlässigkeit für einen landwirtschaftlichen Staub, mit welchem die Staub-Applikatorvorrichtung 1 befüllbar ist. Dies gilt für alle Ausführungsformen der Erfindung. Der Filz, das Filterpapier, sowie das Gewebe können optional zusätzlich staubdurchlässig-perforiert werden, falls sie nicht oder nicht ausreichend staubdurchlässig sind für den jeweiligen landwirtschaftlichen Staub, welcher für die Behandlung von Allergien geeignet ist, mit dem die Staub-Applikatorvorrichtung 1 bzw. deren Staubaufnahme- und Freisetzeinrichtung 2, hier das Säckchen 10 als Aufnahmekammer, befüllbar ist.

Das Säckchen 10 kann dabei, wie zuvor beschrieben, mit einer Einmalfüllung aus dem landwirtschaftlichen Staub 11 durch seine wenigstens eine Befüllöffnung befüllbar sein oder alternativ mit dem landwirtschaftlichen Staub wiederbefüllbar durch seine wenigstens eine Befüllöffnung ausgebildet sein. Das staubdurchlässige oder staubdurchlässig-perforierte Material oder die staubdurchlässige oder staubdurchlässig-perforierte Materialkombination des Säckchens ist beispielsweise aus wenigstens einem Naturmaterial, wenigstens einem Kunststoffmaterial und/oder wenigstens einem Metall oder Metalllegierung. Beispielsweise kann das Säckchen 10 aus Cellulose, Baumwolle, Seide, Leinen, Viskose, Polyester, Polyacryl, Metallfäden zur Bereitstellung eines Metallgewebes, Polyethylen (PE), Polyethylenterephthalat (PET), Polyvinylchlorid (PVC) usw. hergestellt sein. Die Erfindung ist auf die genannten Beispiele von Materialien und Materialkombinationen jedoch nicht beschränkt. Dies gilt für alle Ausführungsformen der Erfindung.

In dem in den Figuren 1 bis 3 gezeigten Ausführungsbeispiel ist das Säckchen 10 aus wenigstens einer Lage aus einem staubdurchlässigen Material 12 hergestellt, beispielsweise aus einem Stoffgewebe usw.. Das Säckchen 10 bildet einen Hohlraum, der mit einem landwirtschaftlichen Staub 11 durch die wenigstens eine Befüllöffnung befüllbar ist.

Des Weiteren kann das Säckchen 10 auf einer Seite, einer Oberseite oder Staubaustrittsseite 13, mit wenigstens einer Lage aus einem staubdurchlässigen oder staubdurchlässig-perforierten Material oder einem staubdurchlässigen oder staubdurchlässig-perforierten Materialkombination ausgebildet sein. Auf der anderen Seite, einer Unterseite oder Befestigungsseite 7, kann das Säckchen 10 wiederum aus wenigstens einer Lage aus einem staubundurchlässigen oder nicht-perforierten Material oder einer staubundurchlässigen oder nicht-perforierten Materialkombination hergestellt sein oder, wie in dem Ausführungsbeispiel in Figur 4 gezeigt ist, aus einem anderen staubdurchlässigen oder staubdurchlässig-perforierten Material oder staubdurchlässigen Materialkombination oder staubdurchlässig-perforierten Materialkombination. Beispielsweise kann das Säckchen 10 an seiner Unterseite oder nicht als Staubaustrittsstelle vorgesehenen Seite 7 aus wenigstens einer nicht-perforierten Kunststofffolie, nicht-perforierten Gummischicht, einer staubundurchlässigen oder nicht-perforierten Papierschicht und/oder nicht-perforierten Metallfolie usw. hergestellt sein. Auf diese Weise ist der landwirtschaftliche Staub 11 nur über die Staubaustrittsseite 13 des Säckchens 7 nach außen aus der Staub-Applikatorvorrichtung 1 heraus ausgebbar.

In einer weiteren Variante kann das Säckchen 10 mit wenigstens einem zusätzlichen im Inneren des Säckchens 10 liegenden Säckchen 10* ausgebildet sein, wie in Figur 2 mit einer gestrichelten Linie angedeutet ist. Dabei kann ein landwirtschaftlicher Staub 11 in dem Hohlraum des innenliegenden Säckchens 10* und/oder in den Hohlraum zwischen dem innenliegenden Säckchen 10* und dem außenliegenden Säckchen 10 vorgesehen werden. Dazu weist neben dem Säckchen 10 auch das innenliegende Säckchen 10* wenigstens eine Befüllöffnung (nicht dargestellt) auf, wenn das innenliegende Säckchen 10* ebenfalls mit einem landwirtschaftlichen Staub 11 befüllbar vorgesehen ist. Es können in dem Säckchen 10 somit ein oder mehrere Säckchen 10* ineinander geschachtelt vorgesehen werden, die mehrere Hohlräume bilden, wobei in wenigstens einem, mehreren oder allen Hohlräumen ein landwirtschaftlicher Staub 11 aufnehmbar ist und die Säckchen 10, 10* zum Befüllen mit dem Staub jeweils wenigstens eine Befüllöffnung hierfür aufweisen.

Das außenliegende Säckchen 10 und das jeweils innenliegende Säckchen 10* sind aus einem, beispielsweise demselben, staubdurchlässigen oder staubdurchlässig-perforierten Material oder staubdurchlässigen oder staubdurchlässig-perforierten Materialkombination zumindest auf ihrer Oberseite bzw. Staubaustrittsseite 13 ausgebildet, derart dass ein in die Säckchen 10, 10* eingefüllter landwirtschaftliche Staub der Staub-Applikatorrvorrichtung 1 nach außen aus dieser und ihrem wenigstens einen oder mehreren Säckchen 10, 10* nach außen heraus abgegeben wird, wie beispielsweise mit den Pfeilen in den Figuren 1, 2 und 4 angedeutet ist. Wenigstens das außenliegende Säckchen 10 kann dabei staubdurchlässig und zusätzlich elastisch oder flexibel ausgebildet sein derart, dass der Hohlraum, den das Säckchen 10 bildet, zusammendrückbar ist und anschließend in seine Ausgangsform oder im Wesentlichen in seine Ausgangsform zurückkehren oder zurückfedern kann für eine aktive, treibmittellose Freisetzung des Staubs 11 aus dem Hohlraum des Säckchens 10.

In Figur 4 ist eine Schnittansicht eines weiteren Ausführungsbeispiels der Staub-Applikatorvorrichtung 1 der Figuren 1 bis 3 gezeigt. Das Säckchen 10 weist dabei auf einer Seite, einer Oberseite oder Staubaustrittsseite 13, wenigstens eine Lage 12 aus einem staubdurchlässigen oder staubdurchlässig-perforierten Material oder einer staubdurchlässigen oder staubdurchlässig-perforierten Materialkombination auf, wobei das jeweilige Material oder die jeweilige Materialkombination wahlweise zusätzlich elastisch oder flexibel ist, derart, dass der durch das Säckchen gebildete Hohlraum zusammendrückbar ist und das Säckchen mit seinem Hohlraum anschließend in seine Ausgangsform zurückkehren oder zurückfedern kann. Auf der anderen Seite, einer Unterseite oder Befestigungsseite 7, weist das Säckchen 10 beispielsweise eine Lage aus einer Schwamm- oder Schaumstoffschicht 14, z.B. einer porösen aber staubundurchlässigen Schwamm- oder Schaumstoffschicht auf. Die Erfindung ist aber auf dieses Beispiel nicht beschränkt. Die Materialien und Materialkombination für die Oberseite und Unterseite des Säckchens 10 sowie die Anzahl der Materiallagen auf der Oberseite und Unterseite können beliebig kombiniert werden, sofern wenigstens eine der Seiten des Säckchens 10 staubdurchlässig oder staubdurchlässig-perforiert ist. Zum Befüllen des Säckchens 10 mit einem landwirtschaftlichen Staub 11, welcher zur Behandlung von Allergien geeignet ist, weist das Säckchen 10 wenigstens eine (nicht dargestellte) Befüllöffnung auf, beispielsweise in der wenigstens einen Lage12 und/oder der Schwamm- oder Schaumstoffschicht 14.

Die wenigstens eine Lage 12 des Säckchens 10 ist staubdurchlässig und, wie zuvor beschrieben, wahlweise zusätzlich elastisch oder flexibel ausgebildet derart, dass der Hohlraum, den das Säckchen 10 mit der wenigstens einen Lage 12 bildet, zusammendrückbar ist und anschließend in seine Ausgangsform oder im Wesentlichen in seine Ausgangsform zurückkehren oder zurückfedern kann für eine aktive, treibmittellose Freisetzung eines in den Hohlraum des Säckchens 10 einfüllbaren landwirtschaftlichen Staubs 11 nach außen. Die Schwamm- oder Schaumstoffschicht 14 ist ebenfalls elastisch oder flexibel ausgebildet derart, dass die Schwamm- oder Schaumstoffschicht 14, wenn sie gedrückt wird, anschließend wieder in ihre Ausgangsform zurückkehren oder zurückfedern kann.

Wie in Figur 4 gezeigt ist, wird ein in dem Säckchen 10 aufgenommener landwirtschaftlicher Staub 11 durch die wenigstens eine Lage 12 aus dem staubdurchlässigen oder staubdurchlässig-perforierten Material oder der staubdurchlässigen oder staubdurchlässig-perforierten Materialkombination auf der Staubaustritts- oder Oberseite 13 nach außen abgegeben. Die Lage aus der Schwamm-oder Schaumstoffschicht 14 hat den Vorteil, dass sie elastisch oder flexibel ist und dadurch beispielsweise die Staub-Applikatorvorrichtung 1 im Gebrauch auch sanft auf die Haut eines Babys oder Kleinkindes auftupfen kann.

In dem in Figur 4 gezeigten Ausführungsbeispiel ist die Staubaufnahme- und Freisetzeinrichtung, hier das Säckchen 10 als Aufnahmekammer mit einem Hohlraum, beispielsweise mit einer Anstecknadel 15 als Befestigungseinrichtung 3 versehen zum Anstecken der Staub-Applikatorvorrichtung 1 z.B. an ein Kleidungsstück, eine Decke oder ein Kissen, z.B. Stillkissen, usw..

Neben einer Anstecknadel 15 oder einer Klebeschicht 6 mit optional zusätzlicher Träger- und Trennschicht 8, wie zuvor in den Figuren 1 bis 3, kann die erfindungsgemäße Staub-Applikatorvorrichtung 1 auch jede andere Befestigungseinrichtung oder jede andere Kombination von Befestigungseinrichtungen aufweisen, die geeignet ist bzw. sind die Staub-Applikatorvorrichtung 1 an einem Gegenstand, insbesondere einem Gefäß wie z.B. eine Babyflasche 4, einem Besteck, z.B. Löffel, wie in Fig. 11 usw., einem Spielzeug, einem Kleidungsstück, einer Decke oder einem Kissen, einem Haushaltsgegenstand usw. zu befestigen. In den nachfolgenden Figuren 5 bis 10 sind z.B. eine Öse, ein Haken und ein Clip als weitere Beispiele von Befestigungseinrichtungen gezeigt. Insbesondere kann die Staub-Applikatorvorrichtung 1 wenigstens zwei Befestigungseinrichtungen, insbesondere wenigstens zwei unterschiedliche Befestigungseinrichtungen, aufweisen, beispielsweise die Anstecknadel 15 in Fig. 4 und ein Gummiband 9 oder eine Klebeschicht 6, wie zuvor in Fig. 1-3 zum Befestigen der Staub-Applikatorvorrichtung 1 an verschiedenen und insbesondere unterschiedlichen Gegenständen, z.B. einem Kissen oder einem Kleidungsstück mittels der Anstecknadel und einer Babyflasche mittels des Gummibands oder der Klebeschicht mit optional zusätzlicher Träger- und Trennschicht usw.. Dies gilt für alle Ausführungsformen der Erfindung. Dabei können die aufgeführten Befestigungseinrichtungen 3 bei der jeweiligen Staub-Applikatorvorrichtung 1 in den gezeigten und beschriebenen Ausführungsformen beliebig miteinander kombiniert werden, je nach Funktion und Einsatzzweck.

In Figur 5 ist eine Perspektivansicht einer Staub-Applikatorvorrichtung 1 gemäß einer dritten Ausführungsform der Erfindung gezeigt. Die Staub-Applikatorvorrichtung 1 weist dabei als eine Staubaufnahme- und Freisetzeinrichtung 2 eine Aufnahmelage mit einer Aufnahmefläche auf der Außenseite, beispielsweise ein Florgewebe 16 oder ein aufgerautes Gewebe auf. Solche Gewebe können aufgrund ihrer Floroberfläche oder aufgerauten Oberfläche als Aufnahmefläche sehr gut mit größeren Mengen an landwirtschaftlichem Staub 11 auf der Außenseite beaufschlagt und anschließend von ihrer durch den Flor oder das Aufrauen vergrößerten Oberfläche aus wieder abgeben werden, insbesondere passiv durch selbstätiges Herunterrieseln, insbesondere im Gebrauch.

Der Begriff Florgewebe ist dabei ein gewebetechnischer Sammelbegriff für alle Gewebe, die eine ein- oder beidseitige Flor- oder Schlingendecke aufweisen. Man unterscheidet zwischen verschiedenen Techniken wie Schusssamt (bzw. Velvet), Cordsamt, Kettsamt, Frottiergewebe oder dem samtähnlichen Effektzwirn, der Chenille. Dieser samtartige Effekt wird nur durch ein Zwei-Fadensystem erreicht. Der Flor bei den Kettflorgeweben, wie beispielsweise Plüsch, Samt, Frottier, Frottiervelours, wird durch eine Flor- oder Polkette erzeugt. Bei Schlingenflor bleiben die Schlingen erhalten, wie beispielsweise Epinglé, während beim Schnittflor die Schlingen dagegen aufgeschnitten sind, wie beispielsweise bei Plüsch, Velours oder Samt.

Zu aufgerauten Geweben gehört beispielsweise Sweatshirt-Stoff. Bei diesem wird auf einer Seite ein sog. Futterfaden eingestrickt, der anschließend aufgeraut wird. Fleece gehört ebenfalls zu den aufgerauten Geweben. Beim Fleece-Stricken entstehen an der Oberfläche kleine Maschen, welche auf einer oder beiden Seiten angeraut werden. Außerdem gehört Nicky-Stoff zu den aufgerauten Geweben. Bei Nicky-Stoff besteht eine Seite aus Polyester und die andere Seite aus langen Baumwollfasern, die aufgeschnitten werden, was die teppichartige Optik erzeugt.

In Figur 5 sind die Fäden 17 des Florgewebes 16, hier z.B. eines Samtgewebes, angedeutet. Diese Florgewebe, wie z.B. Samt, eignen sich sehr gut zur Aufnahme von einem landwirtschaftlichem Staub 11, welcher zur Behandlung von Allergien geeignet ist. Der landwirtschaftliche Staub 11 kann dazu auf die Oberseite, hier Außenseite, oder die Staubaustrittsseite 13, auf das Florgewebe 16 aufgebracht werden. Anschließend löst sich der landwirtschaftliche Staub beim Tragen der Staub-Applikatorvorrichtung 1 von selbst wieder, also passiv, von dem Florgewebe 16 durch selbsttätiges oder selbständiges Herunterrieseln. Statt dem Florgewebe ist es auch möglich eine Klettverschlussoberfläche zu verwenden. Solche Klettverschlussoberflächen weisen festere Kunststofffäden auf als Stofffäden bei einem Florgewebe usw..

In dem in Figur 5 gezeigten Beispiel, weist die Staub-Applikatorvorrichtung 1 beispielsweise als Befestigungseinrichtung 3 eine Öse 18 auf. Eine solche Öse 18 kann leicht an einem Blusen- oder Hemdknopf oder einer Halskette eingehängt werden. Des Weiteren weist die Staub-Applikatorvorrichtung 1 wahlweise zusätzlich ein Trägerelement 19 auf, welches als Träger für die Staubaufnahme- und Freisetzeinrichtung 2, hier die Aufnamelage, z.B. das Florgewebe 16, dient. Die Befestigungseinrichtung 3 und das Trägerelement 19 sind in dem Ausführungsbeispiel in Figur 5 z.B. einstückig aus Kunststoff ausgebildet. Dies ist besonders preisgünstig und einfach in der Herstellung. Die Staubaufnahme- und Freisetzeinrichtung 2, hier die Aufnahmelage, wie z.B. das Florgewebe 16, ist mit seiner Unterseite oder Befestigungsseite auf dem Trägerelement 19 befestigt, z.B. aufgeklebt. Zwischen der Aufnahmelage, hier z.B. dem Florgewebe 16, und dem Trägerelement 19 kann wahlweise zusätzlich wenigstens eine weitere Aufnahmelage vorgesehen werden. Zwischen den beiden Aufnahmelagen kann über wenigstens eine zugeordnete Befüllöffnung wahlweise zusätzlich ein zur Behandlung von Allergien landwirtschaftlicher Staub einfüllbar vorgesehen werden, wobei die Aufnahmelagen hierfür zusätzlich staubdurchlässig und/oder staubdurchlässig perforiert sind. Die wenigstens eine Aufnahmelage oder die Aufnahmelagen können zusätzlich elastisch oder flexibel sein derart, dass die jeweilige Aufnahmelage zusammendrückbar ist und in ihre Ausgangsform zurückfedern oder im Wesentlichen zurückfedern kann, wobei der landwirtschaftliche Staub hierbei nach außen freisetzbar ist. Die Staubaufnahme- und Freisetzeinrichtung 2 kann so als aktive, treibmittellose Staubaufnahme- und Freisetzeinrichtung 2 ausgebildet sein.

Des Weiteren ist in dem in Figur 5 gezeigten Beispiel der landwirtschaftliche Staub 11 auf die Staubaufnahme- und Freisetzeinrichtung 2, hier z.B. das Florgeweben 16 als Aufnahmelage, aufgebracht und die Staub-Applikatorvorrichtung 1 oder zumindest das Trägerelement 19 mit der Staubaufnahme- und Freisetzeinrichtung 2 wahlweise in einer zusätzlichen Schutzhülle 20 aufnehmbar, z.B. aus Papier oder Kunststofffolie. Nachdem die Staubaufnahme- und Freisetzeinrichtung 2 mit einem landwirtschaftlichen Staub 11 beaufschlagt wurde, kann sie mit der Schutzhülle 20 von einem Benutzer versehen und zunächst geschützt werden, um später erst vor Ort zum Gebrauch und damit zum Freisetzen des landwirtschaftlichen Staubs, entfernt zu werden, so dass kein landwirtschaftlicher Staub unterwegs verloren geht.

Ebenso kann, wie in nachfolgender Figur 9 mit einer gestrichelten Linie angedeutet ist, ein abnehmbares Deckelelement 21 vorgesehen sein, das über die mit dem landwirtschaftlichen Staub 11 beaufschlagbare Staubaufnahme- und Freisetzeinrichtung 2, hier die wenigstens eine Aufnahmelage, z.B. das Florgewebe 16, aufbringbar ist. Das Deckelelement 21 ist derart ausgebildet, z.B. an der Staub-Applikatorvorrichtung 1 und beispielsweise deren Trägerelement 19 oder Befestigungseinrichtung 3 lösbar befestigen zu werden, beispielweise durch Aufschrauben, Aufclipsen oder Verrasten usw.. Das Deckelelement 21 kann zudem zusätzlich auch als Staubspeicher für weitere Anwendungen vorgesehen werden. Die Staub-Applikatorvorrichtung 1 muss mit dem Deckelement 21 dazu vor dem Lösen der Staubaufnahme- und Freisetzeinrichtung 2 von dem Deckelelement 21 auf den Kopf gestellt werden, so dass beim Lösen, der verbleibende landwirtschaftliche Staub 11 für weitere Anwendungen nicht aus dem Deckelelement 21 ungewollt herausfallen kann.

In Figur 6 ist eine Staub-Applikatorvorrichtung 1 gemäß einer vierten Ausführungsform der Erfindung in einer Vorderansicht und in Figur 7 in einer Schnittansicht gezeigt.

Bei diesem Ausführungsbeispiel weist die Staub-Applikatorvorrichtung 1 ein mit landwirtschaftlichem Staub 11 befüllbares Gehäuse 22 als Aufnahmekammer mit einem Hohlraum auf. Das Gehäuse ist beispielsweise ein Kunststoffgehäuse, insbesondere ein starres bzw. formstabiles oder alternativ derart flexibles oder elastisches Kunststoffgehäuse, das es mit seinem Hohlraum zusammendrückbar und anschließend wieder in seine Ausgangsform zurückfederbar oder zurückkehrbar ist. Das Gehäuse 22 als Staubaufnahme- und Freisetzeinrichtung 2 ist an wenigstens einer Gehäuseseite mit Staubaustritts-Öffnungen 23 ausgebildet. Das Gehäuse 22 weist beispielsweise wenigstens eine nicht dargestellte Befüllöffnung auf zum Befüllen des Gehäuses 22 mit einem landwirtschaftlichen Staub, wobei die Befüllöffnung wahlweise zusätzlich wiederverschließbar ausgebildet ist. Des Weiteren weist die Staub-Applikatorvorrichtung 1 wenigstens eine zusätzliche Befestigungseinrichtung 3 auf, die mit dem Gehäuse 22 verbunden ist, beispielsweise einen Befestigungshaken 24, welcher mit dem Gehäuse 22 z.B. einstückig ausgebildet ist. Dies ist besonders einfach in der Herstellung und kostengünstig.

Alternativ kann das Gehäuse 22 auch mit einem nicht gezeigten aufklappbaren Gehäusedeckel ausgebildet sein zum Befüllen mit landwirtschaftlichem Staub 11, welcher zur Behandlung von Allergien geeignet ist. Der Gehäusedeckel kann dabei über ein Scharnier schwenkbar mit dem Gehäuse 22 zusätzlich verbunden sein zum Öffnen und Schließen des Gehäuses 22. Wahlweise zusätzlich kann der Gehäusedeckel mit dem Gehäuse 22 verrastbar oder verclipsbar ausgebildet sein, so dass der Gehäusedeckel sich nicht ungewollt öffnen kann. Das Gehäuse 22 mit dem Gehäusedeckel, dem Scharnier, sowie der wenigstens einen Befestigungseinrichtung 3 kann z.B. einstückig aus Kunststoff hergestellt sein. Dies ist besonders einfach und kostengünstig.

Als eine treibmittellose, aktive Staubaufnahme- und Freisetzeinrichtung 2 ist das Gehäuse 22, sowie das Gehäuse mit zusätzlichen Gehäusedeckel, für den eingefüllten Staub staubdurchlässig mittels der wenigstens einen Staub-Austrittsöffnung 23 und zusätzlich elastisch oder flexibel ausgebildet derart, dass das Gehäuse 22 mit seinem Hohlraum zusammendrückbar ist und anschließend in seine Ausgangsform oder im Wesentlichen in seine Ausgangsform zurückkehren oder zurückfedern kann. Ein in das Gehäuse 22 eingefüllter Staub 11 kann durch das Zusammendrücken des Gehäuses 22 und des von ihm gebildeten Hohlraums durch die wenigstens eine Staub-Austrittsöffnung 23 des Gehäuses nach außen aktiv und treibmittellos freigesetzt werden.

In Figur 8 ist eine Staub-Applikatorvorrichtung 1 gemäß einer fünften Ausführungsform der Erfindung in einer Perspektivansicht, in Figur 9 in einer teilweise geschnittenen Seitenansicht und in Figur 10 in einer weiteren, teilweise geschnittenen Seitenansicht gezeigt.

Die Staub-Applikatorvorrichtung 1 weist dabei als Staubaufnahme- und Freisetzeinrichtung 2 z.B. wenigstens eine Aufnahmelage mit einer Aufnahmefläche aus einer Schwamm- oder Schaumstoffschicht 14 auf. Diese hat den Vorteil, dass sie durch ihre poröse Struktur eine große Aufnahmeoberfläche auf ihrer Außenseite zur Aufnahme von landwirtschaftlichem Staub 11, welcher zur Behandlung von Allergien geeignet ist, bereitstellt. Die Schwammschicht 14 kann dabei neben porös auch zusätzlich staubdurchlässig oder staubdurchlässig-perforiert sein oder ebenso nur poröse und staubundurchlässig sein. Dies gilt für alle in der Beschreibung der Erfindung genannten Schwamm- oder Schaumstoffschichten 14. Die wenigstens eine Aufnahmelage, hier z.B. die Schwamm-oder Schaumstoffschicht 14 ist des Weiteren derart elastisch oder flexibel, dass sie zusammengedrückt und anschließend in ihre ursprüngliche Form zurückkehren oder zurückfedern kann, wobei ein auf die Aufnahmefläche auf der Außenseite der Schwamm-oder Schaumstoffschicht aufgebrachter landwirtschaftlichen Staub 11 zusätzlich aktiv, treibmittellos freigesetzt werden kann.

Des Weiteren ist die Staub-Applikatorvorrichtung 1 mit wenigstens einer zusätzlichen Befestigungseinrichtung 3 versehen, welche hierbei beispielsweise einen Clip bzw. Befestigungsclip 25 als Befestigungseinrichtung aufweist. Mittels des Clips 25 kann die Staub-Applikatorvorrichtung 1 insbesondere sehr einfach beispielsweise an Stoff, z.B. Kleidung 5, einer Babydecke, einem Besteck, z.B. Löffel, einem Spielzeug, z.B. Plüschtier usw., befestigt werden. Beispielsweise kann eine Mutter beim Stillen ihres Babys die Staub-Applikatorvorrichtung 1 leicht an ihrer Kleidung oder einer Decke oder einem Stillkissen usw. anclipsen.

Des Weiteren weist die Staub-Applikatorvorrichtung 1 wahlweise zusätzlich ein Trägerelement 19 auf, welches als Träger für die Staubaufnahme- und Freisetzeinrichtung 2, hier z.B. die wenigstens eine Aufnahmelage, hier die wenigstens eine Schwamm- oder Schaumstoffschicht 14, dient. Die Befestigungseinrichtung 3 oder ein Teil der Befestigungseinrichtung, hier ein Teil des Clips 25, und das Trägerelement 19 sind in dem Ausführungsbeispiel in den Figur 8, 9 und 10 z.B. einstückig aus Kunststoff ausgebildet. Dies ist preisgünstig und einfach herzustellen. Die Staubaufnahme- und Freisetzeinrichtung 2, hier die wenigstens eine Aufnahmelage, z.B. die wenigstens eine Schwamm- oder Schaumstoffschicht 14, ist mit ihrer Unterseite oder Befestigungsseite 7 auf dem Trägerelement 19 befestigt, z.B. aufgeklebt.

Die Oberseite oder Staubaustrittsseite 13, hier Außenseite, der Staubaufnahme- und Freisetzeinrichtung 2, hier die Außenseite der Aufnahmefläche der Aufnahmelage, z.B. die Außenseite der wenigstens einen Schwamm- oder Schaumstoffschicht 14, ist mit dem landwirtschaftlichen Staub 11 beaufschlagbar. Die Staub-Applikatorvorrichtung 1 kann optional mit einem zusätzlichen Deckelelement 21, 21* versehen werden, wie mit einer gestrichelten Linie in Figur 9 sowie in der Schnittansicht in Figur 10 angedeutet ist. Das Deckelelement 21, 21* ist in den Figuren 9 und 10 dabei jeweils lösbar an der übrigen Staub-Applikatorvorrichtung 1 angebracht, z.B. an dem Trägerelement 19 und/oder an der Befestigungseinrichtung 3, beispielsweise durch Aufrasten, Verclipsen oder Verschrauben usw.. Das Deckelelement 21 in Figur 9 ist dabei beispielsweise geschlossen ausgebildet und weist keine Staubaustritts-Öffnung auf wie das Deckelelement 21* in Figur 10. Das Deckelelement 21 in Figur 9 muss zum Freisetzen des darunter auf die Staubaufnahme- und Freisetzeinrichtung 2, hier die wenigstens eine Aufnahmelage, aufgebrachten landwirtschaftlichen Staubs 11, erst entfernt werden. In Figur 10 weist das Deckelelement 21* dagegen an wenigstens einer Seite Staubaustritts-Öffnungen 23 auf zum Freisetzen oder Abgeben des darunter auf die Aufnahmefläche der Aufnahmelage, hier der Schwamm-oder Schaumstoffschichtelement 14 aufbringbaren landwirtschaftlichen Staubs 11. Das Deckelelement 21* ist durch seine wenigstens eine oder mehrere Staubaustritts-Öffnungen 23 staubdurchlässig und kann wahlweise zusätzlich elastisch oder flexibel ausgebildet sein derart, dass der Hohlraum, den das Deckelelement 21* bildet, zusammendrückbar ist und anschließend in seine Ausgangsform oder im Wesentlichen in seine Ausgangsform zurückkehren oder zurückfedern kann für eine aktive, treibmittellose Freisetzung eines in den Hohlraum des Deckelelements 21* auf die Aufnahmelage, hier Schwamm- oder Schaumstoffschicht 14, aufbringbaren landwirtschaftlichen Staubs 11.

In Fig. 11 ist ein Ausführungsbeispiel zum Vorsehen der erfindungsgemäßen Staub-Applikatorvorrichtung 1 an einem Löffel 27, als Beispiel für ein Besteck, gezeigt. Die Darstellung in Fig. 11 des Löffels 27 mit seinem Stiel 28, seiner Zunge 30 und seiner Laffe bzw. Kelle 27 ist dabei rein schematisch, stark vereinfacht und nicht maßstäblich.

Als Staub-Applikatorvorrichtung 1, welche in Fig. 11 mit einer gepunkteten Linie angedeutet ist, kann jede der zuvor mit Bezug auf die Fig. 1-10 beschriebenen Staub-Applikatorvorrichtungen 1 vorgesehen werden. Die Staub-Applikatorvorrichtung 1 in Fig. 11 kann dabei wenigstens eine der ebenfalls zuvor mit Bezug auf die Fig. 1-10 beschriebenen Befestigungseinrichtungen 3 aufweisen, sowie zusätzlich oder alternativ eine nachfolgend beschriebene Magnetbefestigungseinrichtung, um z.B., wie in Fig. 11 an dem Besteck, hier Löffel 27 befestigt zu werden. Dies gilt für alle Ausführungsformen der Erfindung.

Die erfindungsgemäße Staub-Applikatorvorrichtung 1, wie sie zuvor beispielhaft mit Bezug auf die Fig. 1 bis 10 beschrieben wurde, kann beispielsweise an einem Besteck, wie dem Löffel 27, z.B. Löffel 27, mit ihrer wenigstens einen Befestigungseinrichtung 3 befestigt werden, wobei ihre wenigstens eine Befestigungseinrichtung 3 dabei beispielsweise die Klebeschicht 6 mit optionaler Träger- und Trennschicht 8, wie zuvor in den Fig. 1-2, das Band, insbesondere Gummiband 9, wie zuvor in Fig. 3, die Befestigungsöse 18, wie zuvor in Fig. 5 zum Einführen des Löffelstiels 28, der Befestigungshaken 24 wie zuvor in den Fig. 6-7 zum Einhängen z.B. an der Zunge 30, der Befestigungsclip 25, wie zuvor in Fig. 8-10 oder eine nachfolgend beschriebenen Magnetbefestigungseinrichtung usw. ist. Dies gilt für alle Ausführungsformen der Erfindung.

Die Anstecknadel 15 als Befestigungseinrichtung 3 der erfindungsgemäßen Staub-Applikatorvorrichtung 1 in Fig. 4 eignet sich dagegen mehr für Stoffe wie bei Kleidungsstücken, Kissen und Decken usw., ist aber grundsätzlich auch als Befestigungseinrichtung 3 zum Befestigung an einem Besteck denkbar, wenn auch nicht bevorzugt.

Das Besteck, hier der Löffel 27, kann beispielsweise im Bereich der Zunge 30 oder an einer anderen Stelle des Stiels 28 mit einer zusätzlichen Vertiefung 29, wie mit einer gestrichelten Linie z.B. in Fig. 11 angedeutet ist, oder auch umgekehrt mit einer Erhebung (nicht dargestellt) ausgebildet sein, an welcher die Staub-Applikatorvorrichtung 1, gemäß der Fig. 1-10 mit ihrer wenigstens einen Befestigungseinrichtung 3 befestigbar ist, z.B. aufklebbar ist mittels der Klebeschicht 6, aufclipsbar ist mittels des Befestigungsclips 25 usw.. Bei einem Löffel, kann das Baby mit seinem Gesicht und insbesondere seiner Nase so in die unmittelbare Nähe der Staub-Applikatorvorrichtung 1 gebracht werden, wenn diese im Bereich der Zunge 30 und dabei in der optionalen Vertiefung 29 oder Erhebung befestigt ist. Das Baby kann dabei ggf. die Staub-Applikatorvorrichtung 1 sogar zusätzlich direkt kontaktieren. Auf diese Weise kann das Baby mittels der Staub-Applikatorvorrichtung 1 mit dem freisetzbaren zur Behandlung von Allergien geeigneten landwirtschaftlichen Staub in Kontakt gebracht werden und den durch die Staub-Applikatovorrichtung freizusetzenden Staub insbesondere einatmen.

Denkbar ist auch, dass die Staub-Applikatorvorrichtung 1 mit integral oder einstückig mit dem Besteck, hier dem Löffel, einem Messer oder einer Gabel verbunden ausgebildet ist. Beispielweise kann die Straub-Applikatorvorrichtung 1 bereits an dem Besteck, hier dem Löffel 27, dem Messer oder der Gabel mit einer Befestigungseinrichtung wie einem Klebstoff, z.B. Schmelzkleber, angeklebt sein oder mit dem Löffel ausgeformt sein. Beispielsweise ist es möglich, dass die Staub-Applikatorvorrichtung 1 in Fig. 7 mit ihrem Gehäuse 22 einstückig mit dem Besteck, hier z.B. Löffel 27, z.B. als Kunststoffteil ausgebildet sind. In diesem Fall braucht es keinen zusätzlichen Befestigungshaken 24 der an dem Gehäuse 22 ausgebildet ist, wie in dem Beispiel in Fig. 7. Jede der anderen Staub-Applikatorvorrichtungen 1 in den Fig. 10 kann ebenfalls einstückig oder integral mit dem Besteck, hier z.B. Löffel ausgebildet sein. Ebenso kann die Staub-Applikatorvorrichtung 1 auf beispielsweise in die Vertiefung 29 in dem Besteck, z.B. Löffel wie in Fig. 11, eingeklemmt und damit bereits an dem Besteck befestigt werden.

Die zuvor mit Bezug auf die Figuren 1 bis 10 beschriebenen Staub-Applikatorvorrichtungen 1 und ihre Befestigungseinrichtungen 3 können untereinander beliebig kombiniert werden. So kann jede der Staub-Applikatorvorrichtungen 1, wie sie in den Fig. 1-10 gezeigt sind, wenigstens eine Befestigungseinrichtung 3 aufweisen wie beispielsweise ein Gummiband 9, wie zuvor in Figur 3, ein mittels Klettverschluss befestigbares Band, ein Befestigungsclip 25 wie zuvor in den Figuren 8 bis 10, eine Anstecknadel 15 wie zuvor in Figur 4, ein Ansteck- oder Befestigungshaken 24 wie zuvor in den Figuren 6 und 7, eine Befestigungsöse 18 wie zuvor in Figur 5, eine nicht dargestellte Schlaufe z.B. aus einem Stoffband usw. zum Einhängen an einem Blusenkopf, einer Kette, oder einem Nuckel des Babyfläschchens usw., eine Klebeschicht 6 mit optionaler Träger- und Trennschicht 8 wie zuvor in den Figur 1 bis 3 und/oder eine nicht dargestellte Magnetbefestigungseinrichtung sein, welche z.B. aus wenigstens einem oder zwei Magnetelementen besteht. Ein erstes Magnetelement ist dabei an der Staubaufnahme- und Freisetzeinrichtung oder an dem Trägerelement befestigt und das zweite Magnetelemente kann zur Befestigung der Staub-Applikatorvorrichtung 1 an einem Kleidungsstück 5 derart angeordnet werden, dass das Kleidungsstück 5 zwischen dem ersten und zweiten Magnetelement angeordnet ist und die beiden Magnetelemente sich dabei gegenseitig ausreichend anziehen, so dass die Staub-Applikatorvorrichtung 1 an dem Kleidungsstück mittels der Magnetbefestigungseinrichtung gehalten werden kann. Ebenso kann die Magnetbefestigungseinrichtung auch nur ein Magnetelement aufweisen zum Befestigen an einer geeigneten Metallflasche, beispielsweise einer geeigneten Thermoskanne aus Metall oder einem geeigneten Metall-Besteck, z.B. Metall-Löffel, Metall-Gabel oder Metall-Messer.

Die Staubaufnahme- und Freisetzeinrichtungen in den Figuren 1 bis 10 und ihre Material(ien), Materialkombination(en), Befestigungseinrichtungen, Trägerelemente, Deckelelemente, Konfiguration, Anzahl der Aufnahmelagen, Anzahl und Position der Befüllöffnungen, Anzahl und Position der Staubaustrittsöffnungen und/oder Schutzhüllen usw. können beliebig variiert, miteinander kombiniert oder ausgetauscht werden, je nach Funktion und Einsatzzweck. Des Weiteren kann die Staub-Applikatorvorrichtung, wie sie zuvor mit Bezug auf die Fig. 1-11 beschrieben wurde auch direkt an einem Schnuller oder einem Beruhigungssauger an dessen Schild, vorzugsweise auf der Außenseite oder alternativ z.B. der Innenseite des Schilds, wo auch das Mundteil sitzt, mittels der Befestigungseinrichtung 3, z.B. der Klebeschicht mit optionaler Träger- und Trennschicht 8 usw., oder durch Verrasten oder Verclipsen usw. befestigt werden oder integral auf der Außenseite oder Innenseite des Schilds mit dem Schnuller oder Beruhigungssauger ausgebildet sein. Das Schild ist dabei das Teil, an welchem das Mundteil bzw. der Sauger des Schnullers oder Beruhigungssaugers befestigt ist. Das Mundteil ist dabei auf der Innenseite des Schilds vorgesehen und die Staub-Applikatorvorrichtung auf der Außenseite des Schilds. Das Schild verhindert dabei das Verschlucken des Saugers. Beispielweise kann die Straub-Applikatorvorrichtung 1 bereits an dem Schild, z.B. der Außenseite oder Innenseite des Schilds mit einer Befestigungseinrichtung wie einem Klebstoff, z.B. Schmelzkleber, angeklebt sein oder mit dem Schild ausgeformt sein. Beispielsweise ist es möglich, dass die Staub-Applikatorvorrichtung 1 in Fig. 7 mit ihrem Gehäuse 22 einstückig mit dem Schild z.B. als Kunststoffteil ausgebildet ist. In diesem Fall braucht es keinen zusätzlichen Befestigungshaken 24 der an dem Gehäuse 22 ausgebildet ist, wie in dem Beispiel zuvor in Fig. 7. Jede der anderen Staub-Applikatorvorrichtungen 1, wie zuvor in den Fig. 1-10 beschrieben, kann ebenfalls einstückig oder integral mit dem Schild ausgebildet sein, sowie in weiteren Ausführungsformen mit einem Gefäß oder einer Babyflasche integral oder einstückig ausgebildet sein. In diesem Fall braucht die Staub-Applikatorvorrichtung 1 nicht unbedingt eine zusätzliche Befestigungseinrichtung 3.

Des Weiteren können beispielsweise die Staub-Applikatorvorrichtung in den Figuren 1, 2, 3 und 5 mit einem zusätzlichen Trägerelement zum Tragen derer Staubaufnahme- und Freisetzeinrichtung ausgebildet sein. Umgekehrt können beispielsweise die Staub-Applikatorvorrichtung in den Figuren 5 bis 10 ohne ein zusätzliches Trägerelement zum Tragen derer Staubaufnahme- und Freisetzeinrichtung und nur mit wenigstens einer Befestigungseinrichtung ausgebildet sein.

Die mit Bezug auf die Figuren 1 bis 11 beschriebenen Ausführungsbeispiele der erfindungsgemäßen Staub-Applikatorvorrichtung sind miteinander kombinierbar, insbesondere einzelne Merkmale.

### Bezugszeichenliste

- 1: Staub-Applikatorvorrichtung
- 2: Staubaufnahme- und Freisetzeinrichtung
- 3: Befestigungseinrichtung
- 4: Babyflasche
- 5: Kleidungsstück
- 6: Klebeschicht
- 7: Befestigungsseite
- 8: Trennschicht
- 9: Gummiband
- 10, 10*: Säckchen
- 11: Landwirtschaftlicher Staub
- 12: Lage aus staubdurchlässigem Material
- 13: Staubaustrittsseite
- 14: Schwamm- oder Schaumstoffschicht
- 15: Anstecknadel
- 16: Florgewebe
- 17: Fäden des Florgewebes
- 18: Befestigungsöse
- 19: Trägerelement
- 20: Schutzhülle
- 21, 21*: Deckelelement
- 22: Gehäuse
- 23: Staubaustritts-Öffnung
- 24: Befestigungshaken
- 25: Befestigungsclip
- 26: Besteck
- 27: Kelle bzw. Laffe
- 28: Löffelstiel
- 29: Vertiefung
- 30: Zunge

## Patentansprüche

1. Staub-Applikatorvorrichtung (1), welcher derart ausgebildet ist insbesondere einen aus einem landwirtschaftlichen Betrieb gewonnenen landwirtschaftlichen Staub, welcher zur Behandlung von Allergien geeignet ist, aufzunehmen und anschließend freizusetzen zur Applikation des landwirtschaftlichen Staubs an Babys, Kleinkinder, Heranwachsende und Erwachsene aufweisend:
eine Staubaufnahme- und Freisetzeinrichtung (2) zum Aufnehmen und Freisetzen eines Staubes,
wobei die Staubaufnahme- und Freisetzeinrichtung (2) zum Freisetzen mit dem landwirtschaftlichen Staub (11) beaufschlagbar ist und wenigstens eine Aufnahmekammer mit einem Hohlraum zum Aufnehmen des landwirtschaftlichen Staubs (11) aufweist, wobei die Aufnahmekammer für den in die Aufnahmekammer einfüllbaren Staub, insbesondere landwirtschaftlichen Staub, staubdurchlässig ausgebildet ist für ein Herausrieselen des Staubs aus der Aufnahmekammer,
und/oder wobei die Staubaufnahme- und Freisetzeinrichtung (2) wenigstens eine Aufnahmelage mit einer Aufnahmefläche auf der Außenseite zum Aufnehmen des landwirtschaftlichen Staubs (11) aufweist derart, dass zumindest der auf der Außenseite der Aufnahmefläche aufbringbare landwirtschaftliche Staub, von der Aufnahmelage herausrieselbar ist,
wobei die Staub-Applikatorvorrichtung (1) wenigstens eine Befestigungseinrichtung (3) aufweist oder die Staub-Applikatorvorrichtung integral oder einstückig mit einem Besteck (27), einem Gefäß, einem Babyfläschchen (4), einem Spielzeug, einem Schnuller oder einem Beruhigungssauger ausgebildet ist.

2. Staub-Applikatorvorrichtung nach Anspruch 1, wobei die Aufnahmekammer ein Gehäuse (22) mit einem Hohlraum aufweist, welches wenigstens eine oder mehrere Staubaustrittsöffnung (23) aufweist, und/oder
wobei die Aufnahmekammer wenigstens ein oder mehrere ineinander geschachtelte Säckchen (10, 10*) mit einem jeweiligen Hohlraum aufweist, wobei das jeweilige Säckchen (10, 10*) staubdurchlässig ausgebildet ist und insbesondere aus einem staubdurchlässigen Material, einer staubdurchlässigen Materialkombination, einem staubdurchlässig-perforierten Material und/oder einer staubdurchlässig-perforierten Materialkombination hergestellt ist oder diese aufweist.

3. Staub-Applikatorvorrichtung nach Anspruch 2, wobei die Staubaufnahme- und Freisetzeinrichtung als aktive, treibmittellose Staubaufnahme- und Freisetzeinrichtung ausgebildet ist (2), wobei das Gehäuse (22) oder das jeweilige Säckchen (10, 10*) elastisch oder flexibel ausgebildet ist, derart, dass das Gehäuse (22) mit seinem Hohlraum bzw. das jeweilige Säckchen (10, 10*) mit seinem Hohlraum zusammendrückbar ist und in seine Ausgangsform zurückfedern oder im Wesentlichen zurückfedern kann, wobei der in dem Hohlraum des Gehäuses (22) bzw. in dem Hohlraum des Säckchens (10, 10*) einfüllbare landwirtschaftliche Staub dabei aus dem Gehäuse (22) bzw. dem Säckchen (10, 10*) nach außen freisetzbar ist.

4. Staub-Applikatorvorrichtung nach einem der vorstehenden Ansprüche, wobei unter der Aufnahmelage wenigstens eine weitere Aufnahmelage vorgesehen ist, wobei zwischen den beiden Aufnahmelagen zusätzlich ein Staub, insbesondere landwirtschaftlicher Staub, einbringbar ist und wobei die Aufnahmelagen hierzu für den zwischen die beiden Aufnahmelagen einbringbaren landwirtschaftlichen Staub zusätzlich staubdurchlässig und/oder staubdurchlässig perforiert sind derart, dass der Staub nach außen austreten kann.

5. Staub-Applikatorvorrichtung nach einem der vorstehenden Ansprüche, wobei wenigstens eine Aufnahmelage aus einem Stoffgewebe, einem Metallgewebe, wenigstens einer Papierschicht, wenigstens einer Folienschicht, wenigstens einer Filterpapierschicht, und/oder wenigstens einer Schwamm- oder Schaumstoffschicht (14) besteht.

6. Staub-Applikatorvorrichtung nach einem der vorstehenden Ansprüche, wobei die Staubaufnahme- und Freisetzeinrichtung (2) als aktive, treibmittellose Staubaufnahme- und Freisetzeinrichtung (2) ausgebildet ist, wobei die Aufnahmelage oder Aufnahmelagen elastisch oder flexibel sind derart, dass die jeweilige Aufnahmelage zusammendrückbar ist und in ihre Ausgangsform zurückfedern oder im Wesentlichen zurückfedern kann, wobei der landwirtschaftliche Staub hierbei nach außen freisetzbar ist.

7. Staub-Applikatorvorrichtung nach einem der vorstehenden Ansprüche, wobei die wenigstens eine Befestigungseinrichtung (3) zum Befestigen der Staub-Applikatorvorrichtung (1) an einem Gefäß, einem Babyfläschchen (4), einem Besteck (27), einem Spielzeug, einem Schnuller, einem Beruhigungssauger, einem Kleidungsstück (5), einer Decke und/oder einem Kissen, insbesondere Stillkissen, ausgebildet ist.

8. Staub-Applikatorvorrichtung nach einem der vorstehenden Ansprüche, wobei die Befestigungseinrichtung (3) wenigstens eine Klebeschicht (6), eine Klebeschicht mit einer abziehbaren Träger- und Trennschicht (8), eine Anstecknadel (15), eine Befestigungsöse (18), ein Befestigungshaken (24), ein Befestigungsclip (25), ein Band (9), eine Lasche, oder eine Magnetbefestigungseinrichtung ist.

9. Staub-Applikatorvorrichtung nach einem der vorstehenden Ansprüche, wobei die Staub-Applikatorvorrichtung (1) ein Trägerelement (19) als Träger für die Staubaufnahme- und Freisetzeinrichtung (2) aufweist, wobei das Trägerelement (19) einstückig mit der Befestigungseinrichtung (3) ausgebildet ist oder die Befestigungseinrichtung (3) als separates Teil an dem Trägerelement (19) befestigt ist, insbesondere angeklebt.

10. Staub-Applikatorvorrichtung nach einem der Ansprüche 2 bis 9, wobei wenigstens ein Bereich, insbesondere wenigstens eine Seite (7, 13) des jeweiligen Säckchens (10, 10*) aus wenigstens einer Lage aus einem für den einfüllbaren landwirtschaftlichen Staub staubdurchlässigen oder staubdurchlässig-perforierten Material oder einer für den landwirtschaftlichen Staub staubdurchlässigen oder staubdurchlässig-perforierten Materialkombination besteht zur Freisetzung des landwirtschaftlichen Staubs (11) nach außen aus der Staub-Applikatorvorrichtung (1).

11. Staub-Applikatorvorrichtung nach einem der Ansprüche 2 bis 10, wobei das Gehäuse (22) einen Gehäusedeckel aufweist zum Öffnen und Schließen des mit dem landwirtschaftlichen Staub (11) beaufschlagbaren Hohlraums des Gehäuses (22), wobei der Gehäusedeckel vollständig abnehmbar oder über wenigstens ein Scharnier schwenkbar mit dem Gehäuse (22) verbunden ausgebildet ist und wobei der Gehäusedeckel insbesondere geschlossen ausgebildet oder mit wenigstens einer oder mehreren Staubaustritts-Öffnungen (23) versehen ist.

12. Staub-Applikatorvorrichtung nach Anspruch 9, wobei die Staub-Applikatorvorrichtung (1) ein Deckelelement (21, 21*), insbesondere ein teilweise oder vollständig abnehmbares Deckelelement (21, 21*), aufweist zum Abdecken zumindest der Staubaufnahme- und Freisetzeinrichtung (2), wobei das Deckelelement (21, 21*) geschlossen oder mit einer oder mehreren Staubaustritts-Öffnungen (23) versehen ist und wobei das Deckelelement (21, 21*) formstabil oder derart elastisch oder flexibel ausgebildet ist, dass es zusammendrückbar und in seine Ausgangsform zurückfederbar oder im Wesentlichen zurückfederbar ist.

13. Staub-Applikatorvorrichtung nach einem der vorstehenden Ansprüche, wobei wenigstens die Staubaufnahme- und Freisetzeinrichtung (2) mit einer Schutzhülle (20) versehbar ist, wobei die Schutzhülle (20) insbesondere aus Kunststofffolie und/oder Papier ist.

14. Staub-Applikatorvorrichtung nach einem der vorstehenden Ansprüche, wobei die Staub-Applikatorvorrichtung als separates Teil an einem Besteck (27), einem Gefäß, einem Babyfläschchen (4), einem Spielzeug, einem Schnuller oder einem Beruhigungssauger befestigbar ist, insbesondere mittels der Befestigungseinrichtung (3) oder einem Kleber, insbesondere Schmelzkleber.

15. Staub-Applikatorvorrichtung nach einem der vorstehenden Ansprüche, wobei das Besteck ein Löffel (27), ein Messer oder eine Gabel ist, wobei der Löffel vorzugsweise eine zusätzliche Vertiefung (29) oder Erhebung für die Verbindung mit der Staub-Applikatorvorrichtung (1) aufweist, wobei die Staub-Applikatorvorrichtung (1) integral oder einstückig mit dem Löffel (27) verbunden ist oder als separates Teil mit dem Löffel (27) verbunden ist mittels der wenigstens einen Befestigungseinrichtung (3) der Staub-Applikatorvorrichtung (1).
